(19) Europäisches Patentamt
European Patent Office
Office européen des brevets

(11) Publication number: **0 367 463 A1**

(12) # EUROPEAN PATENT APPLICATION

(21) Application number: 89310859.7

(51) Int. Cl.5: C07K 7/10 , A61K 37/02

(22) Date of filing: 20.10.89

The title of the invention has been amended (Guidelines for Examination in the EPO, A-III, 7.3).

(30) Priority: 20.10.88 PCT/GB88/00877
20.10.88 GB 8824615
20.10.88 GB 8824618
20.10.88 GB 8824619

(43) Date of publication of application:
09.05.90 Bulletin 90/19

(84) Designated Contracting States:
AT BE CH DE ES FR GB GR IT LI LU NL SE

(71) Applicant: CELLTECH LIMITED
216 Bath Road
Slough Berkshire SL1 4EN(GB)

(72) Inventor: Eaton, Michael Anthony William
Nethercote Chinnor Road
Ashton Rowant Oxfordshire OX9 5SH(GB)
Inventor: Beeley, Nigel Robert Arnold
16, Cheshire Road
Thame Oxfordshire OX9 3LQ(GB)
Inventor: Bose, Christopher Cyril
23, Collection Drive
Twyford Reading Berkshire RC10 0AU(GB)

(74) Representative: Hallybone, Huw George et al
CARPMAELS AND RANSFORD 43
Bloomsbury Square
London WC1A 2RA(GB)

(54) **Calcitonin gene related peptide analogues.**

(57) Novel analogues of calcitonin gene related peptide comprise analogues of natural CGRPs modified at at least two of positions 3, 22 and 25. These compounds are of general formula
$NH_2$-Ala-Cys-A-$aa^{\alpha}$-B-C-Lys-D-$aa^{\beta}$-$CONH_2$
wherein
A is a carboxylate or amide substituted amino acid or amino acid analogue;
$aa^{\alpha}$ is substantially the amino acid sequence of a CGRP from position 4 to position 21;
B is a hydrocarbyl or thio substituted amino acid or amino acid analogue;
C is valine or glycine;
D is an amine or hydroxyl substituted amino acid or amino acid analogue.

The novel analogues may comprise natural amino acid residues. Preferably A is aspartate or asparagine, B is valine or methionine, C is valine and D is asparagine or serine. The analogues exhibit desirable cardiovascular properties and are potentially useful in therapy, e.g. for treatment of deficiencies in cerebral blood supply.

EP 0 367 463 A1

# CHEMICAL COMPOUNDS

## Field of the Invention

This invention relates to novel analogues of calcitonin gene-related peptide and to processes for their production and to their therapeutic use.

## Background of the Invention

Calcitonin gene-related peptide (CGRP) is a product of the calcitonin gene system. Alternative processing of RNA transcribed from the calcitonin gene leads to the production in neuronal tissue of CGRP, a 37 amino acid peptide. CGRP has been discovered in a number of species including rats, chickens and humans. The CGRP family of compounds are very closely related differing from each other by no more than a few amino acids. Human CGRP exists as α-CGRP (see British Patent No. GB 2141430B) β-CGRP (see European Patent Application No. EP-A-188400), and the existence of γ-CGRP has also been reported (Westermark et al, (1986) Biochem and Biophys. Res. Commun. 140 (3) 827-831).

The amino acid sequence of human α-CGRP is:
ACDTATCVTH RLAGLLSRSG GVVKNNFVPT NVGSKAF
using the well known single letter code to designate amino acid residues.

The principal use that has been described for CGRP is use in the treatment of hypertension in view of its effects on the cardiovascular system where it has been found to cause vasodilatation and to lower blood pressure. CGRP has also been postulated to play a role in calcium regulation and gastric secretion. Recently CGRP has been reported to be useful in the treatment of deficiencies in cerebral blood supply (see our copending published International Patent Application WO 89/03686).

We have investigated analogues of natural CGRPs and have identified a group of novel peptide analogues derived from CGRP which exhibit useful biological activity. The compounds are CGRP analogues modified at at least two of positions 3, 22 and 25.

## Summary of the Invention

Accordingly the present invention provides compounds of Formula I

I $NH_2$-Ala-Cys-A-$aa^{\alpha}$-B-C-Lys-D-$aa^{\beta}$-$CONH_2$

wherein Ala, Cys and Lys denote Alanine, Cysteine and Lysine amino acid residues respectively;
A represents an aspartate amino acid residue or a structure of Formula II.

II

COR
$(CH_2)n$
-NH-CH-CO-

wherein n is an integer from 1 to 6; R is a hydroxyl group or the group $NR_1R_2$ where $R_1$ and $R_2$ are the same or different and are hydrogen atoms or straight or branched $C_{1-6}$ alkyl groups, cycloalkyl groups, e.g. $C_{3-8}$ cycloalkyl groups, aryl groups, e.g. $C_{6-12}$ aryl groups, or cycloalkyl alkyl groups, e.g. cyclopropyl methyl groups;
$aa^{\alpha}$ represents substantially the amino acid sequence of a CGRP from position 4 to position 21;
B represents a valine amino acid residue or a structure of Formula III.

III

$R_3$
-NH-CH-CO-

wherein $R_3$ represents a hydrocabyl group other than isopropyl or the group $(CH_2)_m$-$SCH_3$ wherein m is an integer from 1 to 6;
C represents a Valine or Glycine amino acid residue;
D represents an asparagine amino or glutamine acid residue or a structure of Formula IV.

IV

$R_4$-CH-OH
$(CH_2)_p$
-NH-CH-CO-

wherein p is 0 or an integer from 1 to 6; $R_4$ is a hydrogen atom, a straight or branched $C_{1-5}$ alkyl group, a cycloalkyl group such as a $C_{3-8}$ cycloalkyl group, an aryl group such as a $C_{6-12}$ aryl group e.g. phenyl, an aralkyl group e.g. an ar$C_{1-3}$ alkyl group or a cycloalkylalkyl group;
$aa^{\beta}$-$CONH_2$ represents substantially the sequence of a CGRP from position 26 to position 37;
and the salts and protected derivatives thereof provided that when A is an aspartate residue B is not a valine residue and D is not an asparagine residue, that when B is a valine residue A is not an aspartate residue and D is not an asparagine residue, and that when D is an asparagine residue, A is not an aspartate residue and B is not a valine residue.

It will be appreciated, of course, that the letters A, C and D do not represent the amino acid residues Alanine, Cysteine and Aspartate.

The compounds of Formula I are analogues of CGRP which are modified at at least two of positions 3, 22 and 25 with respect to the amino acid sequence of human $\alpha$-CGRP. We have found that analogues having such modifications exhibit desirable in vivo cardiovascular activity in rat experiments. These experimental results indicate that the analogues have potential for use in cardiovascular therapy; for example for use in the treatment of deficiencies in cerebral blood supply as described in our copending published International Patent Application WO 89/03686.

When $R_3$ is a hydrocarbyl group, in the compounds of Formula I, it may be an aliphatic, cycloaliphatic, or aromatic hydrocarbyl group. $R_3$ may be, for example, an alkyl group such as a straight or branched chain $C_{1-5}$ alkyl group, a cycloalkyl group such as a $C_{3-8}$ cycloalkyl group, an aryl group such as a $C_{6-12}$ aryl group e.g. phenyl, an arakyl group e.g. an ar$C_{1-3}$ alkyl group such as a benzyl, phenethyl or indolemethyl group, or a cycloalkylalkyl group e.g. a cyclopropylmethyl group.

R,$R_1$, $R_2$, $R_3$ or $R_4$ may, if desired, be substituted by, for example, straight or branched chain alkyl groups e.g. a $C_{1-16}$ alkyl group.

In particular embodiments, however, the residues A, B and D of the compounds of Formula I are natural amino acid residues. Thus A may be an aspartate, glutamate, asparagine or glutamine residue, and is preferably an aspartate or asparagine residue. B may be a glycine, analine, valine, proline, leucine, isoleucine, phenylalanine, tyrosine, tryptophan, cysteine or methionine residue. Preferably, B is a valine or methionine residue. D may be an asparagine, glutamine, serine or threonine residue, and is preferably an asparagine or serine residue. Preferably, also, C is a valine residue.

As used herein the term 'substantially the sequence' denotes a sequence which is substantially homologous, within the relevant region, to a naturally occurring CGRP. Such naturally occurring CGRPs include in particular rat, human, or chicken $\alpha$,$\beta$ or $\gamma$ CGRP. The sequence preferably shows greater than 90% homology with a CGRP, and is more preferably greater than 95% homologous to a naturally occurring CGRP. Most preferably the sequence is identical to that of a naturally occurring CGRP, e.g. human $\alpha$ CGRP.

It will be understood that where compounds of Formula (I) possess two or more cysteine residues, e.g. at positions 2 and 7, these are typically the form of a disulphide bridge.

Salts include acid addition salts e.g. inorganic acid salts such as those formed with mineral acids e.g. hydrochloric acid, sulphuric acid or phosphoric acid and salts formed with bases e.g. alkali metal salts such as sodium, potassium, magnesium or calcium salts and ammonium salts formed with ammonia or suitable organic amines.

The salts are preferably pharmaceutically acceptable salts.

Particular embodiments of the compounds of Formula I which have been synthesised and found to have desirable cardiovascular properties are the compounds hereinafter referred to as CB0008, CB0011 and H7030. The amino acid sequences of these compounds are given hereinafter in the specific examples.

The compounds according to the invention can be prepared by known methods. They may be prepared

by chemical synthesis or where appropriate by recombinant DNA technology.

The peptides according to the invention are most conveniently prepared using standard solid phase and or liquid phase peptide synthesis techniques (see for example Merrifield R. B. Fed. Proc. Fed. Amer. Soc. Exp. Biol, 24, 412 (1962)). In such solid phase synthesis, the solid phase support acts as a C-terminal protecting group for the growing oligomer chain.

Thus, in general, for solid phase synthesis, N-terminal protected amino acid or peptide is reacted with a suitably functionalised insoluble polymer such that the C-terminal residue is attached to the insoluble support. The N-terminal protecting group is then selectively removed from the aminoacyl polymer and the next N-protected amino acid or peptide or derivative e.g. amino acid analogue is coupled to the polymer using a suitable reagent for activation of the carboxyl group of the amino acid or peptide to be introduced. This cycle of deprotection and coupling can then be repeated as necessary, using the appropriate amino acid, peptide or derivative, to assemble on the polymer carrier the desired sequence of the peptide of Formula (1). Once the sequence is complete, a more vigorous reagent is applied to the peptide-polymer, to cleave the bond linking the peptide to the polymer, thus liberating the peptide which can be recovered using conventional techniques.

Depending on conditions and/or resin support used for solid phase synthesis used, the peptide obtained may have C-terminal acid or amide group and may, or may not, possess an N-terminal protecting group.

It will also be appreciated that any other reactive group(s) such as amino, carboxy, hydroxy or mercapto group(s) if present, will have been suitably protected during the synthesis any may still be in a protected state after cleavage of the peptide from the polymer. Further processing of the peptide is therefore often necessary to obtain the desired compound of Formula (1), as described hereinafter, particularly, for example, for the introduction of disulphide bridges. The introduction and removal of protective groups is well known in the art (see for example "The Peptides" Volume 3 ed. Gross and Meienhoffer, Academic Press 1981).

The amino acid or peptide starting materials, or reactive derivatives thereof for use in the solid phase synthesis are either known compounds, or may be prepared by methods analogous to those used for the preparation of the known compounds.

Particular reagents which may be used for activation of the carboxyl group of the amino acid or peptide include for example imides such as dicyclohexylcarbodiimide, pentafluorophenyl esters or the amino acid anhydrides themselves.

The resin may be for example an insoluble polymeric support e.g. a polyamide resin such as a cross-linked polydimethylacrylamide resin or any inert macroreticular resin such as polystyrene cross-linked with divinylbenzene or a methylbenzhydrylamine resin.

In particular we have found there are two procedures which are useful in the preparation of compounds of the invention. The first of these is the BOC procedure where the protecting group used in the synthetic cycle is a tertiary butoxycarbonyl group. The BOC protecting group is selectively removed at each stage using trifluoracetic acid and dichloromethane. After completion of the synthetic cycles side chain protecting groups may be removed from the peptide by treatment with hydrogen fluoride and anisole. The second procedure is known as the FMOC procedure and utilises a fluorenylmethoxycarbonyl group which is selectively removed using 20% piperidine in dimethylformamide. Side chain protecting groups are removed from the peptide by treatment with trifluoroacetic acid and anisole. The peptide is removed from the resin by treatment with methanol and ammonia.

Where compounds of Formula (I) possess an intra- or inter-molecular disulphide bridge the final step in the synthesis will be the formation of the bridge. Thus a compound which has the primary sequence of a compound of Formula (I) and which contains at least two cysteine residues, may be oxidised to introduce a disulphide bridge and provide the end product.

The oxidation may be effected using any oxidising agent capable of oxidising two cysteine residues to a disulphide. Air oxidation or oxidation in oxygenated aqueous solution may be used. Suitable oxidising agents include thallium trifluoroacetate. The starting materials for this reaction may be obtained by the solid phase synthesis described above after the removal of any mercapto protecting groups which may be present using standard techniques.

The C-terminal amide group of compounds of Formula (I) may be introduced by appropriate choice of the cleavage conditions used in the solid phase synthesis described above. Thus, the compound may be cleaved from the support and amidated in a one step process by treatment with, for example methanol and ammonia.

Alternatively, where the cleavage conditions are chosen to yield a peptide with a C-terminal carboxylic acid the required amide may be obtained by conventional means, including enzymatic treatment. For example, a 1-38 residue peptide intermediate having a C-terminal leucine residue may be converted to the compound

of Formula I using carboxypeptidase Y and ammonia. Alternatively, a 1-38 residue peptide intermediate having a C-terminal glycine residue may be converted using the α amidating enzyme as described by Bradbury A. F. et al (Nature 298 240-244 (1982)). Also compounds of Formula I may be prepared by chemical treatment of activated derivatives of the 1-37 residue peptide with for example ammonia.

Where the compound of formula I has naturally occurring amino acids at all of the positions 3, 22 and 25, the compounds may be prepared via recombinant DNA technology. The DNA sequence coding for said compounds of Formula (I) may be chemically synthesised using methods well known in the art such as by using the phosphotriester and phosphite procedures (see for example, "Polymer - Supported Synthesis of Oligonucleotides" by M. J. Gait in "Polymer - Supported Reactions in Organic Synthesis" Eds P. Hodges and D. C. Sheerington John Wiley & Sons Ltd (1980) 435-456).

Also DNA sequences coding for naturally occurring CGRPs may be obtained in tissues producing the CGRPs, by the following procedure. cDNA clone banks may be made from mRNA prepared from mammalian tissues. The gene encoding the CGRP may then be identified by probing the cDNA banks with label led DNA probes based on an N-terminal consensus sequence for CGRP. Provided appropriate hybridisation conditions are used, the labelled probes will hybridise to the DNA sequence coding for the CGRP. The DNA sequence may be sequenced using standard procedures.

DNA sequence encoding the compound of Formula I may be constructed from such cloned DNA sequences by appropriate restriction enzyme digestion and ligation of DNA fragments. The DNA sequence which codes for the compound of Formula I may then be inserted in a suitable expression vector and expressed using techniques well known in the art, for example as described in Maniatis et al (1982) "Molecular Cloning - A Laboratory Manual" Cold Spring Harbour Laboratory.

The host cell used for cloning and expression may be any eukaryotic cell such as for example plant or insect cells, yeast cells e.g. S. cerevisiae or a mammalian cell such as for example CHO cells or cells of myeloid origin e.g. myeloma or hybridoma cells. The host cell may be any prokaryotic cell such as for example a gram negative bacterium e.g. E. coli; a gram positive bacterium e.g. a species of Bacillus such as B. subtilis or a species of Streptomyces e.g. S. lividans.

Suitable hosts and vectors for cloning and expression include those described in British Patent No. GB 2141430B for use with human α-CGRP and include particularly the dual origin vectors described in British Patent No. GB 2136814B. The use of recombinant DNA technology yields the corresponding C-terminal carboxylic acids of Formula (I). The C-terminal amide group may be introduced using conventional techniques such as by treatment with a suitable enzyme or by chemical treatment as described above to yield the desired compound of Formula (I).

Peptides of Formula (I) have been found to be effective in selectively increasing blood flow through the carotid arteries, and are believed therefore to be of use in the treatment of diseases associated with a reduced blood flow to the brain such as subarachnoid haemorrhage, cerebral stroke and migraine. They may also be of use in treatment of cardiac disorders and hypertension.

Thus in a second aspect the invention provides a therapeutic composition comprising a compound of Formula (I) in combination with a suitable pharmaceutically acceptable diluent, excipient or carrier.

Accordingly also in a third aspect the invention provides a method of therapeutic treatment comprising administering an effective amount of a compound of Formula I to a human or animal subject.

The compounds may be used therapeutically in any of the circumstances in which use of CGRP is appropriate including treatment of deficiencies in cerebral blood supply; for example, as described in our copending published International Patent Application WO 89/03686.

In order to be useful in the treatment of deficiencies in cerebral blood supply it is essential that the therapeutic agent is able to selectively affect the cerebrovascular bed, such that the blood supply is increased at the desired site. A further desirable property for the therapeutic agent is that the blood supply should be increased without substantially lowering the blood pressure. Current therapeutic strategies to reverse constriction of cerebral blood vessels are unsatisfactory because they also result in lowering of blood pressure, so exacerbating the original problem. Hitherto, therefore, there has been a real need for an effective treatment for restoring cerebral blood flow and/or reversing cerebral vasospasm. We have found that it is possible to achieve both the required selectivity of site of action and the desired increase in cerebral blood supply without substantially affecting blood pressure, by administration of an appropriate amount of a CGRP analogue of the invention. Also we have found, when using CGRP-based compounds, blood flow to vital organs, such as the brain, e.g. internal carotid blood flow, is increased even when a significant hypotensive effect is observed.

Furthermore in a fourth aspect the invention provides a process for the production of a pharmaceutical composition according to the invention comprising admixing a compound of Formula I with a pharmaceutically acceptable carrier, excipient or diluent.

Pharmaceutical compositions for use according to the present invention may be formulated in conventional manner, optionally with one or more physiologically acceptable carriers diluents or excipients.

Compounds for use according to the present invention may be formulated for oral, buccal, parenteral or rectal administration or in a form suitable for nasal administration or administration by inhalation or insufflation.

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. sodim lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents; emulsifying agents, non-aqueous vehicles; and preservatives. The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The CGRP analogue may be formulated for parenteral administration by injection, e.g. by bolus injection or continuous infusion. Formulations for injection may be presented in unit dosage form. The compositions may take such forms as suspensions, solutions or emulsions in oily or aqueous vehicles, and may contain formulatory agents such as suspending, stabilising and/or dispersing agents.

Alternatively, the active ingredient may be in powder form for constitution with a suitable vehicle, e.g. sterile pyrogen-free water, before use.

The CGRP analogue may also be formulated in rectal compositions such as suppositories or retention enemas, e.g. containing conventional suppository bases such as cocoa butter or other glycerides.

In addition to the formulations described previously, the CGRP analogue may also be formulated as a depot preparation. Such long acting formulations may be administered by implantation or by intramuscular injection.

For nasal administration or administration by inhalation, the compounds for use according to the present invention are conveniently delivered in the form of an aerosol spray presentation from pressurised packs or a nebuliser, with the use of a suitable propellant, e.g. dichlorodi-fluoromethane, trichlorofluoromethane, dichlorotetrafluoroethane, carbon dioxide or other suitable gas.

The pharmaceutical compositions may, if desired, be presented in a pack or dispenser device which may contain one or more unit dosage forms containing the active ingredient. The pack or dispenser device may be accompanied by instructions for administration.

When used for treatment of deficiencies in cerebral blood supply the dose at which the CGRP analogue will be administered to man will be such that the cerebral blood supply is differentially increased and preferably blood pressure is not substantially affected. The precise dose of CGRP analogue will depend upon the route of administration, the potency of the analogue and the body weight and pathology of the patient. The important factor is believed to be the concentration of CGRP analogue which is present at the target vascular bed. On an individual patient basis the dose of the CGRP analogue which should be administered to cause the desired effect may be determined by administering a low dose for 10-20 minutes and then increasing the dose every 10-20 minutes until the desired effect is seen. A CGRP analogue may be administered to an average 70kg man by IV infusion at doses in the range 0.01-32ng/kg/min, preferably in the range 0.06 to 24ng/kg/min, more preferably in the range 4 to 24ng/kg/min, and most preferably in the range 4-16ng/kg/min. For example, the analogue may be administered to an average 70kg man by IV infusion at doses in the range 4ng/kg/min to 16ng/kg/min over a time period of 20 minutes. In some cases it may be desirable to infuse the patient with the analogue for longer time periods e.g. for up to or greater than 1 hour or for more than 24 hours.

The invention is further illustrated in the following non-limiting examples and with reference to the accompanying diagrams, Figures 1-6, of which Figures 2, 3 and 5 relate to comparative examples.

<u>Brief Description of the Drawings</u>

Figure 1 shows a graph of cardiovascular responses to CGRP analogue CB0011 infusion for 60 min in rats

Figure 2 shows a graph of cardiovascular responses to CGRP analogue CB0010 infusion for 60 min in rats

Figure 3 shows a graph of cardiovascular responses to CGRP analogue CB0009 infusion for 60 min in rats

Figure 4 shows a graph of cardiovascular responses to CGRP analogue CB0008 infusion for 60 min in rats

Figure 5 shows a graph of cardiovascular responses to CGRP analogue CB0007 infusion for 60 min in rats

Figure 6 shows a graph of cardiovascular responses to CGRP analogue H70030 infusion for 60 min in rats

In Figures 1 to 6 the following symbols denote dosage levels as indicated below

▲ = 0.006nmol/hr
○ = 0.06nmol/hr
● = 0.6nmol/hr

Description of Specific Embodiments

Example 1

CGRP analogues CB0007, CB0008, CB0009, CB0010, CB0011 and H7030 have the following sequences:

| | | | | |
|---|---|---|---|---|
| ACDTATCVTH | RLAGLLSRSG | GMVKNNFVPT | NVGSKAF | - CB0007 |
| ACDTATCVTH | RLAGLLSRSG | GMVKSNFVPT | NVGSKAF | - CB0008 |
| SCDTATCVTH | RLAGLLSRSG | GVVKNNFVPT | NVGSKAF | - CB0009 |
| ACDTATCVTH | RLAGLLSRSG | GVVKNNFVPT | NVGSKAF | - CB0010 |
| ACNTATCVTH | RLAGLLSRSG | GMVKNNFVPT | NVGSKAF | - CB0011 |
| ACNTATCVTH | RLAGLLSRSG | GVVKSNFVPT | NVGSKAF | - H70030 |

These CGRP analogues were synthesised using a methylbenzyhydrylamine resin. The peptides were synthesised using the FMOC procedure on an AB430A Peptide Synthesiser using the STDF1 Synthesis File with double couplings at Arginine and Histidine. The FMOC group was removed at the end of the synthesis without resin sampling. Cys-acm groups were used for protection. Ethers and esters were protected as t-butyl esters. Arginine was protected as its MTR ester.

One of these analogues (H7030) was also synthetised by Boc solid phase synthesis procedure as follows:

The synthesis was carried out by solid phase techniques using 3gm. 4 Methylbenzhydrylamine Resin (0.7nmol/gm.). Between 6 m.eq and 10 m.eq of Boc amino acid were used for coupling. The amounts of solvents for washing resin were about 15 ml.-20 ml./gm. of resin (TFA/$CH_2Cl_2$15 ml.-20 ml./gm). After washing Cys coupling, the TFA/$CH_2Cl_2$ used for removal of Boc group was incorporated with DTE (2%).

For cleavage, 10 g. of peptide resin was treated with HF (100 ml.) + Anisole (10 ml.) at 0°C for 1 hour. After removal of HF, the peptide resin was washed with ether and extracted with Acetic acid. The Acetic acid extract was diluted with water and stirred at room temperature until it gave negative Ellman test.

The contents were then purified by HPLC.

Introduction of disulphide Bridge

100mg of the above peptides were added to 1 litre of previously oxygenated $H_2O$ containing 1mM ammonium bicarbonate to pH8.

The mixtures were left at room temperature for 3 hours. 40ml of glacial acetic acid was added to pH2

and the mixture was freeze dried.

The resulting solids were analysed by hplc.

Example 2

Conscious, Wistar rats (n = 8 in all groups) with renal, mesenteric and hindquarters flow probes, or with bilateral common carotoid flow probes, were given CGRP analogues CB0007 and CB0008, or CB0009 and CB0011, or CB0010 and H7030 (structures given in Example 1). The animals were randomised to receive one of each pair of analogues on the first experimental day (the other analogue being given on the second experimental day) at doses of 0.006, 0.06 and 0.6nmol/hr for periods of 1 hour, separated by 1 hour post-infusion periods. The results are shown in Figures 1 to 6.

The CGRP analogues were synthesised using conventional peptide synthesis [see for example Merrifield R.B., Fed. Proc. Fed. Amer. Soc. Exp. Biol. 24 412 (1962)] following the FMOC procedure as described in Example 1.

The experimental protocol ran over 2 days and animals were randomised to receive one of the following pairs of analogues on day 1:

CB0007 or CB0008: CB0009 or CB0011; CB0010 or H7030

On the second experimental day the remaining analogue of the pair was given. All analogues were dissolved in isotonic saline (containing 1% bovine serum albumin); the amount of lyophilizate dissolved was adjusted according to the stated peptide content, such that infusion of appropriately diluted solutions at a rate of 0.3ml/hr would deliver doses calculated to be 0.006, 0.06 or 0.6nmol/hr. Following a 30 min baseline period, infusions were given for 60 min, followed by a 60 min post-infusion period. All analogues were given in increasing doses to avoid any carry-over effects of the higher doses.

Measurements were made at -10, 0,5, 10, 20, 30, 40, 50, 60, 70, 80, 90, 100, 110, and 120 min, with the infusion running between 0 and 60 min.

At a dose of 0.6nmol/hr all analogues caused tachycardia, hypotension and hindquarters and carotid hyperaemias. However, while the changes in heart rate and mean blood pressure were similar with all analogues, 7030 caused the numerically largest carotid hyperaemia, (as judged from the integrated, i.e. area-under-the- curve response) and did so without compromising renal perfusion, although there was a marked reduction in mesenteric blood flow. Comparison with previous data obtained with human $\alpha$- and $\beta$-CGRP indicated that analogue H7030 caused a greater overall carotid hyperaemia than both these peptides at 0.6nmol/hr.

At a dose of 0.06nmol/hr, analogues CB0007 and CB0008 (that were administered to the same animals) exerted significantly different carotid hyperaemic effects, with the latter analogue being more potent. Compound H7030 also had greater carotid hyperaemic effects than CB0007, and both CB0008 and H7030 exerted their enhanced carotid hyperaemic effects without influencing renal blood flow, whereas the latter variable was reduced in the presence of CB0007. Analogue CB0008 also caused a greater increase in carotid blood flow than did analogues CB0009 and CB0010.

At a dose of 0.006nmol/hr, analogue H7030 was the only one to cause an increase in heart rate. At this dose, however, this occurred in the absence of carotid hyperaemia or changes in mean blood pressure or systemic haemodynamics, indicating that compound might have augmented myocardial actions.

The profile of activity of the CGRP analogues showing a selective effect on the carotids in rats supports their use in the treatment of deficiencies in cerebral blood supply in humans.

Comparative effects of CGRP analogues

In order to assess possible differences in the vasodilator effects of the CGRP analogues, the hindquarters and carotid hyperaemic responses to infusions of 0.06 and 0.6nmol/hr were quantitated by measurement of the areas under the response curves given in Figures 1-6. Table 1 shows that, with both infusions, there was a range of responses in the hindquarters, but simultaneous comparison of all six groups showed there were no significant differences between any of them. However, Table 2 shows that there were significant differences between some of the carotid hyperaemic responses, particularly at the 0.06nmol/hr dose. The difference between the responses to analogues CB0007 and CB0008 is notable because these data were obtained from the same animals. The tendency towards a greater response to H7030 than to CB0010 should be registered for the same reason.

Table 1

| Integrated hindquarters hyperaemic responses to CGRP analogues (estimated from the areas under the curves) expressed in arbitrary units; values are mean (SEM) | |
|---|---|
| 0.06nmol/hr | 0.6nmol/hr |
| CB0007 62 (19) | CB0007 163 (27) |
| CB0009 84 (23) | CB0010 184 (27) |
| CB0011 88 (18) | CB0009 185 (31) |
| CB0010 96 (20) | H7030 255 (49) |
| CB0008 118 (27) | CB0008 259 (60) |
| H7030 126 (34) | CB0011 299 (63) |
| The data are ranked in order of response at each dose, although there were no significant differences between the responses (Kruskal-Wallis Test). | |

Table 2

| Integrated hindquarters hyperaemic responses to CGRP analogues (estimated from the areas under the curves) expressed in arbitrary units; values are mean (SEM) | |
|---|---|
| 0.06nmol/hr | 0.6nmol/hr |
| CB0007 118(23)[a,b] | CB0009 717(91)[e] |
| CB0009 162(29)[c] | CB0010 841(103) |
| CB0010 175(48)[d] | CB0007 922(96) |
| CB0011 275(57) | CB0008 1005(117) |
| H7030 334(53) | CB0011 1104(153) |
| CB0008 420(60) | H7030 1300(113) |

a,P 0.05 CB0007 vs. CB0008;
b,P 0.05 CB0007 vs. H7030;
c,P 0.05 CB0009 vs. CB0008;
d,P 0.05 CB0010 vs. CB0008;
e,P 0.05 CB0009 vs. H7030. (Kruskal-Wallis test).
The data are ranked in order of response at each dose.

In summary, however, the results given in Tables 1 and 2 indicate that analogues CB0008, CB0011 and H7030, especially the latter analogue, have particularly desirable properties supporting their use in the treatment of deficiencies in cerebral blood supply. It is to be noted that all of these latter analogues are modified at at least two of positions 3, 22 and 25 with respect to the amino acid sequence of human α-CGRP.

In comparison, analogues CB0007, CB0009 and CB0010 appear to have less desirable properties. It is to be noted that these analogues (CB0007, CB0009 and CB0010) each have only a single amino acid modification with respect to the amino acid sequence of human α-CGRP. CB0007 is modified at residue 22,

CB0009 at residue 1 and CB0010 at residue 35.

## Claims

1. A Compound of Formula I

I

$$NH_2\text{-Ala-Cys-A-aa}^{\alpha}\text{-B-C-Lys-D-aa}^{\beta}\text{-CONH}_2$$

as defined

wherein Ala, Cys and Lys denote Alanine, Cysteine and Lysine amino acid residues respectively;
A represents an aspartate amino acid residue or a structure of Formula II.

II

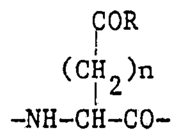

wherein n is an integer from 1 to 6; R is a hydroxyl group or the group $NR_1R_2$ where $R_1$ and $R_2$ are the same or different and are hydrogen atoms or straight or branched $C_{1-6}$ alkyl groups, cycloalkyl groups, e.g. $C_{3-8}$ cycloalkyl groups, aryl groups, e.g. $C_{6-12}$ aryl groups, or cycloalkyl alkyl groups, e.g. cyclopropyl methyl groups;
$aa^{\alpha}$ represents substantially the amino acid sequence of a CGRP from position 4 to position 21;
B represents a valine amino acid residue or a structure of Formula III.

III

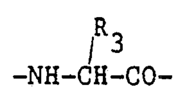

wherein $R_3$ represents a hydrocabyl group other than isopropyl or the group $(CH_2)_m\text{-}SCH_3$ wherein m is an integer from 1 to 6;
C represents a Valine or Glycine amino acid residue;
D represents an asparagine amino or glutamine acid residue or a structure of Formula IV.

IV

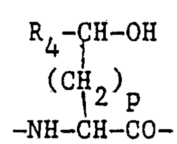

wherein p is 0 or an integer from 1 to 6; $R_4$ is a hydrogen atom, a straight or branched $C_{1-6}$ alkyl group, a cycloalkyl group such as a $C_{3-8}$ cycloalkyl group, an aryl group such as a $C_{6-12}$ aryl group e.g. phenyl, an aralkyl group e.g. an $arC_{1-3}$ alkyl group or a cycloalkylalkyl group;
$aa^{\beta}\text{-CONH}_2$ represents substantially the sequence of a CGRP from position 26 to position 37;
and the salts and protected derivatives thereof provided that when A is an aspartate residue B is not a valine residue and D is not an asparagine residue, that when B is a valine residue A is not an aspartate residue and D is not an asparagine residue, and that when D is an asparagine residue, A is not an aspartate residue and B is not a valine residue.

2. A Compound according to Claim 1 in which A, B, and D are natural amino acid residues.

3. A Compound according to Claim 1 or 2 in which A is an aspartate, glutamate, asparagine or glutamine residue.

4. A Compound according to any of the preceding claims in which B is a glycine, alanine, valine, proline, leucine, isoleucine, phenylalamine, tyrosine, tryptophan, cysteine, or methionine residue.

5. A Compound according to any of the preceding claims in which D is an asparagine, glutamine, serine or threonine residue.

6. A Compound according to any of the preceding claims in which C is valine.

7. A polypeptide according to Claim 1 of amino acid sequence ACDTATCVTH RLAGLLSRSG GMVKSNFVPT NVGSKAF

8. A polypeptide according to Claim 1 of amino acid sequence ACNTATCVTH RLAGLLSRSG GMVKNNFVPT NVGSKAF

9. A polypeptide according to Claim 1 of amino acid sequence ACNTATCVTH RLAGLLSRSG GVVKSNFVPT NVGSKAF

10. A process for preparation of a compound according to Claim 1 comprising chemical synthesis by solid or liquid phase peptide synthesis techniques.

11. A process for the production of a compound according to Claim 2 comprising expressing a DNA sequence which codes for the compound.

12. A therapeutic composition comprising a compound according to Claim 1 in combination with a suitable pharmaceutically acceptable diluent, excipient or carrier.

13. A method of therapeutic treatment comprising administering an effective amount of a compound according to Claim 1 to a human or animal subject.

14. A process for the production of a therapeutic composition comprising admixing a compound according to Claim 1 with a pharmaceutically acceptable carrier, excipient or diluent.

15. The use of a compound according to Claim 1 in the treatment of deficiencies in cerebral blood supply.

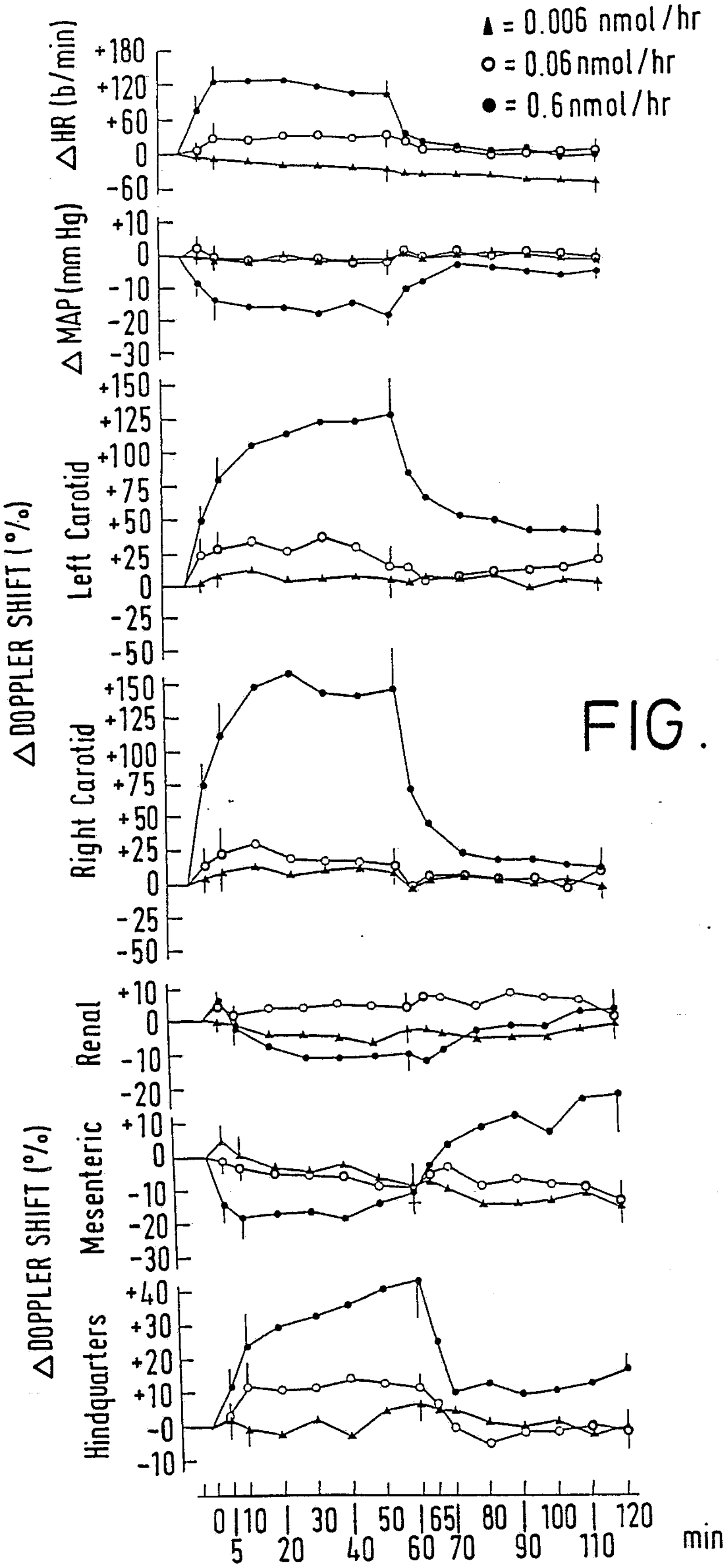
▲ = 0.006 nmol/hr
○ = 0.06 nmol/hr
● = 0.6 nmol/hr
Δ HR (b/min)
+180
+120
+60
0
-60
Δ MAP (mm Hg)
+10
0
-10
-20
-30
Δ DOPPLER SHIFT (%)
Left Carotid
+150
+125
+100
+75
+50
+25
0
-25
-50
Right Carotid
+150
+125
+100
+75
+50
+25
0
-25
-50
Δ DOPPLER SHIFT (%)
Renal
+10
0
-10
-20
Mesenteric
+10
0
-10
-20
-30
Hindquarters
+40
+30
+20
+10
-0
-10
0 10 30 50 65 80 100 120
5 20 40 60 70 90 110
min

FIG. 1

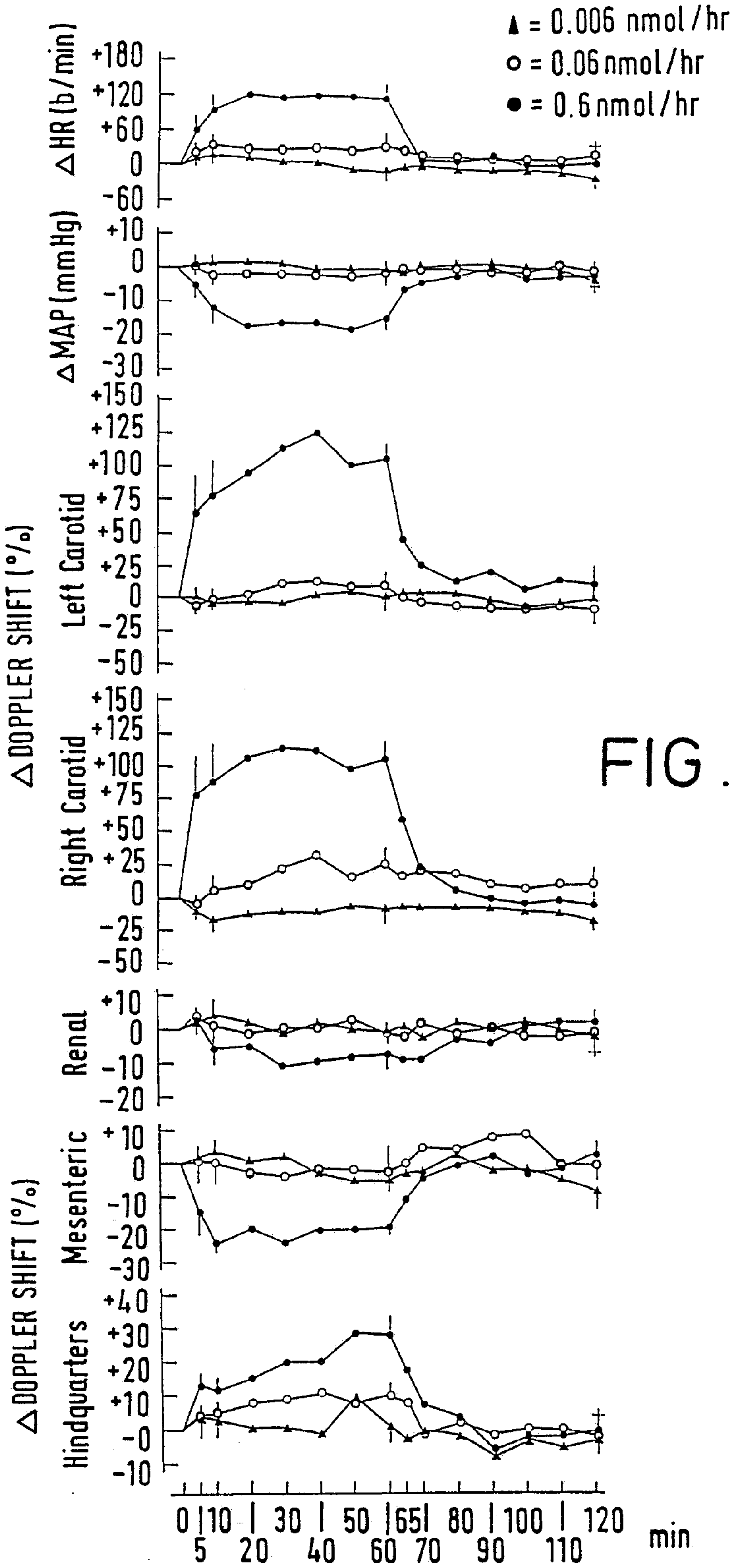
▲ = 0.006 nmol/hr
○ = 0.06 nmol/hr
● = 0.6 nmol/hr
ΔHR (b/min)
ΔMAP (mmHg)
Δ DOPPLER SHIFT (%)
Left Carotid
Right Carotid
Renal
Mesenteric
Hindquarters
min

FIG. 2

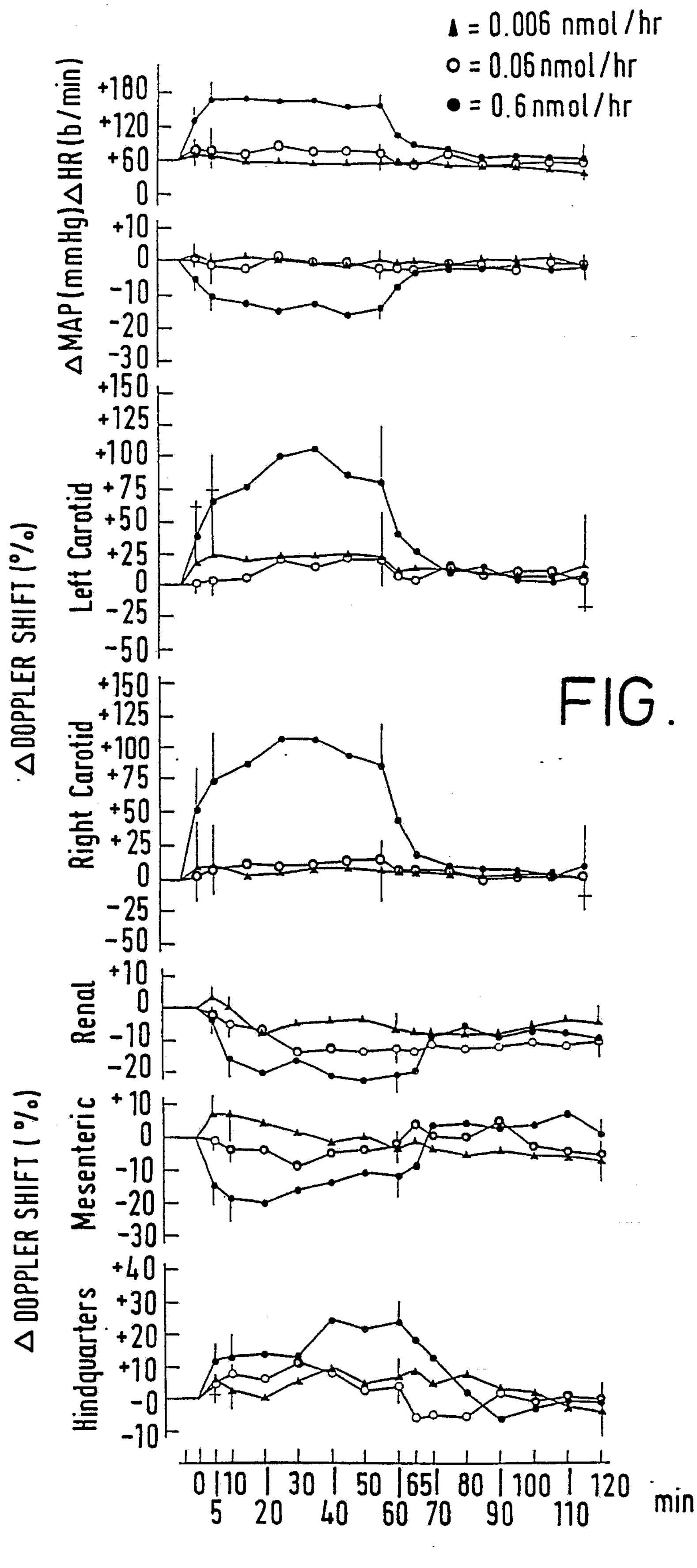
▲ = 0.006 nmol/hr
○ = 0.06 nmol/hr
● = 0.6 nmol/hr
ΔMAP(mmHg) ΔHR(b/min)
ΔDOPPLER SHIFT (%)
Left Carotid
Right Carotid
Renal
Mesenteric
Hindquarters
ΔDOPPLER SHIFT (%)
min

FIG. 3

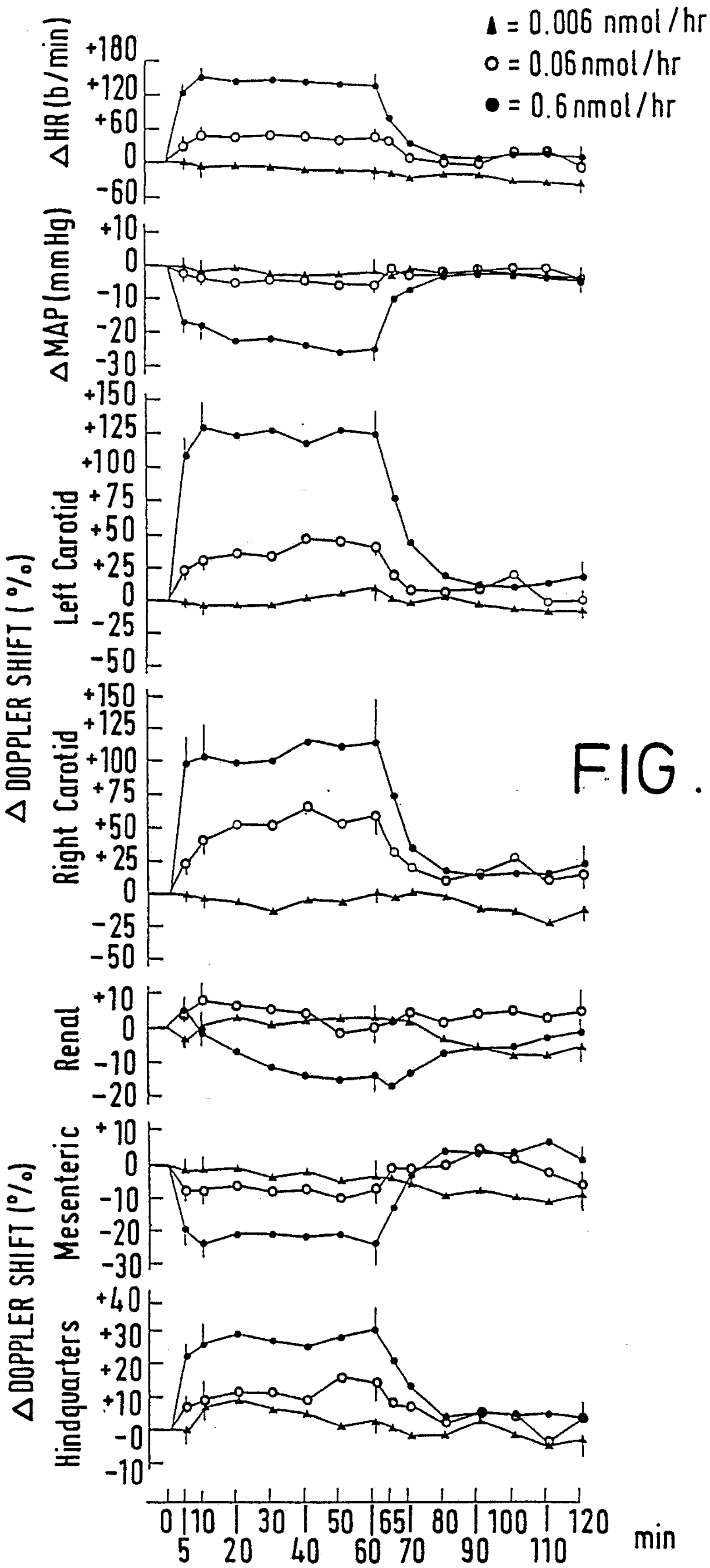
▲ = 0.006 nmol/hr
○ = 0.06 nmol/hr
● = 0.6 nmol/hr
ΔHR (b/min)
+180
+120
+60
0
-60
ΔMAP (mmHg)
+10
0
-10
-20
-30
Δ DOPPLER SHIFT (%)
Left Carotid
+150
+125
+100
+75
+50
+25
0
-25
-50
Right Carotid
+150
+125
+100
+75
+50
+25
0
-25
-50
Renal
+10
0
-10
-20
Δ DOPPLER SHIFT (%)
Mesenteric
+10
0
-10
-20
-30
Hindquarters
+40
+30
+20
+10
-0
-10
0 10 30 50 65 80 100 120
5 20 40 60 70 90 110
min

FIG. 4

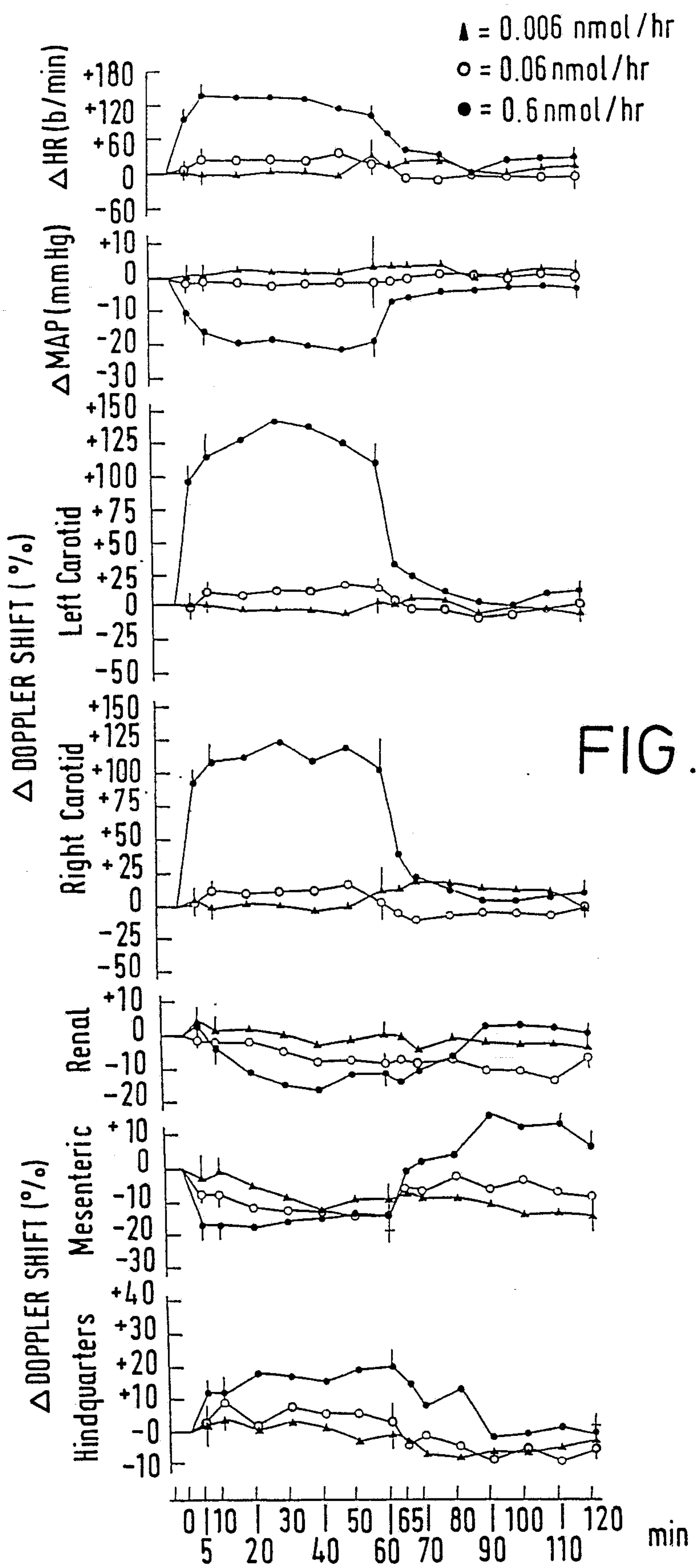
▲ = 0.006 nmol/hr
○ = 0.06 nmol/hr
● = 0.6 nmol/hr
Δ HR (b/min)
+180
+120
+60
0
-60
Δ MAP (mmHg)
+10
0
-10
-20
-30
Δ DOPPLER SHIFT (%)
Left Carotid
+150
+125
+100
+75
+50
+25
0
-25
-50
Right Carotid
+150
+125
+100
+75
+50
+25
0
-25
-50
Δ DOPPLER SHIFT (%)
Renal
+10
0
-10
-20
Mesenteric
+10
0
-10
-20
-30
Hindquarters
+40
+30
+20
+10
-0
-10
0 10 30 50 65 80 100 120
5 20 40 60 70 90 110
min

FIG. 5

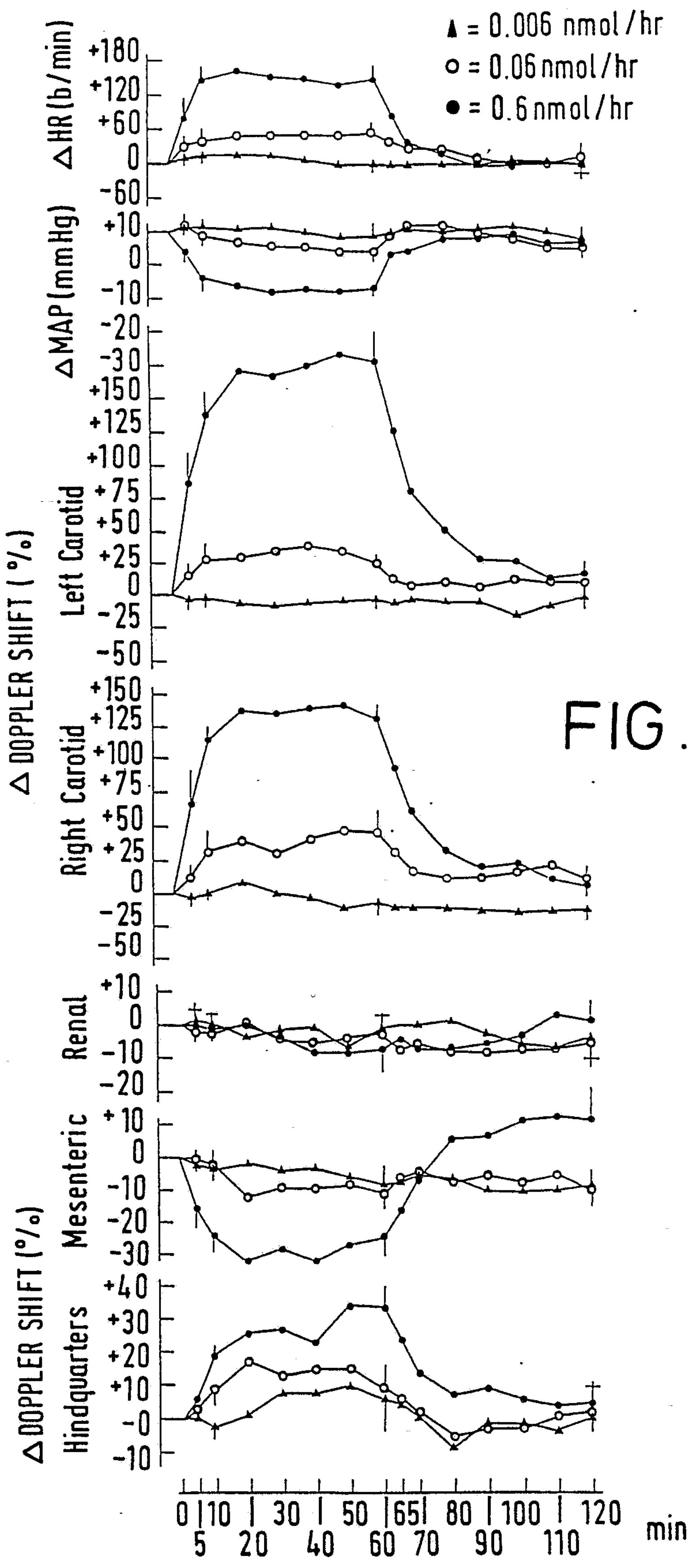
▲ = 0.006 nmol/hr
○ = 0.06 nmol/hr
● = 0.6 nmol/hr
Δ HR (b/min)
+180
+120
+60
0
-60
Δ MAP (mmHg)
+10
0
-10
-20
-30
Δ DOPPLER SHIFT (%)
Left Carotid
+150
+125
+100
+75
+50
+25
0
-25
-50
Right Carotid
+150
+125
+100
+75
+50
+25
0
-25
-50
Δ DOPPLER SHIFT (%)
Renal
+10
0
-10
-20
Mesenteric
+10
0
-10
-20
-30
Hindquarters
+40
+30
+20
+10
-0
-10
0 5 10 20 30 40 50 60 65 70 80 90 100 110 120
min

FIG. 6

European Patent Office

# PARTIAL EUROPEAN SEARCH REPORT

which under Rule 45 of the European Patent Convention shall be considered, for the purposes of subsequent proceedings, as the European search report

Application number
EP 89 31 0859

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A- 212 432 (TOYO JOZO)<br>* Page 3, discussion of background *<br>-- | 1-6 | C 07 K 7/10<br>A 61 K 37/02 |
| X | FEBS LETTER, vol. 183, no. 2, April 1985, pages 403-407, Elsevier Science Publishers B.V., Amsterdam, NL;<br>P.H. STEENBERGH et al.: "A second human calcitonin/CGRP gene"<br>* Page 403, abstract; page 405, figure; page 407, lines 8-17 *<br>-- | 1-6,11 | |
| A | NATURE, vol. 313, January 3 1985, pages 54-56, London, GB;<br>S.D. BRAIN et al.: "Calcitonin gene-related peptide is a potent vasodilator"<br>* Page 54, figure *<br>-- ./. | 1-6 | TECHNICAL FIELDS SEARCHED (Int. Cl.4)<br>C 07 K<br>A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-12

Claims searched incompletely:

Claims not searched: 13

Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 09-01-1990 | KORSNER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application number

EP 89 31 0859



| DOCUMENTS CONSIDERED TO BE RELEVANT | | | CLASSIFICATION OF THE APPLICATION (Int. Cl 4) |
|---|---|---|---|
| Category | Citation of document with indication, where appropriate, of relevant passages | Relevant to claim | |
| D,P, X | WO-A-89 03 686 (CELLTECH)<br>* Page 2, line 28 - page 3, line 9; page 14, lines 1-15; page 15, table 2; claims * | 1-9<br>12,14-15 | |
| | ---- | | |

TECHNICAL FIELDS SEARCHED (Int Cl 4)